# EUROPEAN PATENT APPLICATION

(11) **EP 4 374 898 A1**
(43) Date of publication of application: **29.05.2024**
(21) Application number: 22306728.1
(22) Date of filing: 23.11.2022
(51) Int. Cl.: A61M 5/34

(54) **DRUG DELIVERY DEVICE COMPRISING AN ADAPTOR**

(71) Applicant: Becton Dickinson France, 38800 Le Pont-de-Claix (FR)
(72) Inventor: RIVIER, Cédric, 38340 VOREPPE (FR); THUILLIEZ, Julien, 38240 MEYLAN (FR); LEIBBRAND, Alfred, 38640 Claix (FR); DOLEGA, Lukasz, 38950 St Martin Le Vinoux (FR)
(74) Representative: Germain Maureau

(57) **Abstract**

Provided herein is drug delivery device including: a barrel defining an interior configured to receive a composition, the barrel including a distal tip defining a channel to provide a passageway for transfer of the composition; a cylindrical adaptor mounted onto the distal tip, the adaptor including an internal thread defining at least a portion of an inner surface and an outer surface including at least one plane, the adaptor rotatable relative to the barrel; and a film applied to at least a portion of the barrel and at least a portion of the adaptor, thereby preventing relative rotation between the adaptor and the barrel.

## Description

### BACKGROUND OF THE INVENTION

### Field of the Invention

The present invention is directed to a drug delivery device having an adaptor element mounted thereon and a film to engage with the adaptor to prevent its rotation relative to the rest of the drug delivery device.

### Description of Related Art

When connected to a syringe, it may be desirable to prevent a luer lock adaptor from rotating relative to the syringe. This can be accomplished by heat shrinking a label or other film to both the adaptor and the syringe, for example as set forth in U.S. Patent No. 10,661,018, the disclosure of which is incorporated herein by reference in its entirety. However, such solutions may require heat tunnel equipment, which can increase capital costs and occupy space on a manufacturing floor.

Further, the application of such labels or films to a pre-filled syringe, following filling of the device, may not be desirable, as heat-sensitive compositions should not unnecessarily be exposed to heated conditions. Therefore, there exists a need in the art for a film or label to be applied to an adaptor and a syringe in order to prevent the relative rotation of the adaptor that removes or reduces the need for heat shrinking. There also exists a need to provide a more secure, anti-rotational connection between the adaptor and the syringe regardless of the application method.

### SUMMARY OF THE INVENTION

Provided herein is a drug delivery device including: a barrel defining an interior configured to receive a composition, the barrel including a distal tip defining a channel to provide a passageway for transfer of the composition; a cylindrical adaptor mounted onto the distal tip, the adaptor including an internal thread defining at least a portion of an inner surface and an outer surface including at least one plane, the adaptor rotatable relative to the barrel; and a film applied to at least a portion of the barrel and at least a portion of the adaptor, thereby preventing relative rotation between the adaptor and the barrel.

Also provided herein is a drug delivery device including: a barrel defining an interior configured to receive a composition, the barrel including a distal tip defining a channel to provide a passageway for transfer of the composition; a cylindrical adaptor mounted onto the distal tip, the adaptor including an internal thread defining at least a portion of an inner surface and a smooth outer surface, the adaptor rotatable relative to the barrel; and a film applied to at least a portion of the barrel and at least a portion of the adaptor, thereby preventing relative rotation between the adaptor and the barrel.

Also provided herein is a drug delivery device including: a barrel defining an interior configured to receive a composition, the barrel including a distal tip defining a channel to provide a passageway for transfer of the composition; a cylindrical adaptor including an inner surface and an opposing outer surface, the adaptor mounted onto the distal tip, including an internal thread defining at least a portion of the inner surface, and rotatable relative to the barrel; a film applied to at least a portion of the barrel and at least a portion of the adaptor, thereby preventing relative rotation between the adaptor and the barrel; and an identification tag arranged with the adaptor and beneath the film, where the identification tag is configured to communicate wirelessly with a corresponding reader device.

Also provided herein is a drug delivery system including: the drug delivery device described herein; and a connector including an external surface including a thread, where the connector is engaged with the drug delivery device by the thread of the connector engaged with the internal thread of the adaptor.

Also provided herein is a method of manufacturing a drug delivery device described herein, including: mounting the adaptor on the distal tip of the barrel; and applying the film to the at least a portion of the barrel and the adaptor.

Also provided herein is a method of delivering a composition with a drug delivery device described herein, including: connecting the drug delivery device to a connector including an external surface including a thread, where the connector is engaged with the drug delivery device by the thread of the connector engaged with the internal thread of the adaptor; and causing the composition within an interior of the barrel to flow through the connector.

Other non-limiting embodiments of the invention will be set forth in the following numbered clauses:
Clause 1: A drug delivery device, comprising: a barrel defining an interior configured to receive a composition, the barrel comprising a distal tip defining a channel to provide a passageway for transfer of the composition; a cylindrical adaptor mounted onto the distal tip, the adaptor comprising an internal thread defining at least a portion of an inner surface and an outer surface comprising at least one plane, the adaptor rotatable relative to the barrel; and a film applied to at least a portion of the barrel and at least a portion of the adaptor, thereby preventing relative rotation between the adaptor and the barrel.
Clause 2: The drug delivery device of clause 1, wherein the drug delivery device is sterilized prior to application of the film to the at least a portion of the barrel and the adaptor.
Clause 3: The drug delivery device of clause 1 or 2, wherein the film comprises an inner surface and an opposed outer surface, wherein at least a portion of the inner surface comprises an adhesive, such that when the film is applied to the at least a portion of the barrel and the adaptor, the inner surface of the film is adhered to a surface of the barrel and a surface of the adaptor.
Clause 4: The drug delivery device of any of clauses 1-3, further comprising a plug removably connected to the adaptor.
Clause 5: The drug delivery device of any of clauses 1-4, wherein a diameter of the barrel is substantially the same as a diameter of the adaptor.
Clause 6: The drug delivery device of any of clauses 1-5, wherein the barrel comprises glass.
Clause 7: The drug delivery device of any of clauses 1-6, wherein the outer surface of the adaptor is smooth.
Clause 8: The drug delivery device of any of clauses 1-7, further comprising an identification tag arranged with the adaptor and beneath the film, wherein the identification tag is configured to communicate wirelessly with a corresponding reader device.
Clause 9: The drug delivery device of clause 8, wherein the adaptor is over-molded with the identification tag.
Clause 10: A drug delivery device, comprising: a barrel defining an interior configured to receive a composition, the barrel comprising a distal tip defining a channel to provide a passageway for transfer of the composition; a cylindrical adaptor mounted onto the distal tip, the adaptor comprising an internal thread defining at least a portion of an inner surface and a smooth outer surface, the adaptor rotatable relative to the barrel; and a film applied to at least a portion of the barrel and at least a portion of the adaptor, thereby preventing relative rotation between the adaptor and the barrel.
Clause 11: The drug delivery device of clause 10, wherein the drug delivery device is sterilized prior to application of the film to the at least a portion of the barrel and the adaptor.
Clause 12: The drug delivery device of clause 10 or 11, wherein the film comprises an inner surface and an opposed outer surface, wherein at least a portion of the inner surface comprises an adhesive, such that when the film is applied to the at least a portion of the barrel and the adaptor, the inner surface of the film is adhered to a surface of the barrel and a surface of the adaptor.
Clause 13: The drug delivery device of any of clauses 10-12, further comprising a plug removably connected to the adaptor.
Clause 14: The drug delivery device of any of clauses 10-13, wherein a diameter of the barrel is substantially the same as a diameter of the adaptor.
Clause 15: The drug delivery device of any of clauses 10-14, wherein the barrel comprises glass.
Clause 16: The drug delivery device of any of clauses 10-15, wherein the outer surface of the adaptor comprises a pair of planes diametrically opposed to one another.
Clause 17: The drug delivery device of any of clauses 10-16, further comprising an identification tag arranged with the adaptor and beneath the film, wherein the identification tag is configured to communicate wirelessly with a corresponding reader device.
Clause 18: The drug delivery device of clause 17, wherein the adaptor is over-molded with the identification tag.
Clause 19: A drug delivery device, comprising: a barrel defining an interior configured to receive a composition, the barrel comprising a distal tip defining a channel to provide a passageway for transfer of the composition; a cylindrical adaptor comprising an inner surface and an opposing outer surface, the adaptor mounted onto the distal tip, comprising an internal thread defining at least a portion of the inner surface, and rotatable relative to the barrel; a film applied to at least a portion of the barrel and at least a portion of the adaptor, thereby preventing relative rotation between the adaptor and the barrel; and an identification tag arranged with the adaptor and beneath the film, wherein the identification tag is configured to communicate wirelessly with a corresponding reader device.
Clause 20: The drug delivery device of clause 19, wherein the drug delivery device is sterilized prior to application of the film to the at least a portion of the barrel and the adaptor.
Clause 21: The drug delivery device of clause 19 or 20, wherein the film comprises an inner surface and an opposed outer surface, wherein at least a portion of the inner surface comprises an adhesive, such that when the film is applied to the at least a portion of the barrel and the adaptor, the inner surface of the film is adhered to a surface of the barrel and a surface of the adaptor.
Clause 22: The drug delivery device of any of clauses 19-21, further comprising a plug removably connected to the adaptor.
Clause 23: The drug delivery device of any of clauses 19-22, wherein a diameter of the barrel is substantially the same as a diameter of the adaptor.
Clause 24: The drug delivery device of any of clauses 19-23, wherein the barrel comprises glass.
Clause 25: The drug delivery device of any of clauses 19-24, wherein the outer surface of the adaptor comprises a pair of planes diametrically opposed to one another.
Clause 26: The drug delivery device of any of clauses 19-25, wherein the outer surface of the adaptor is smooth.
Clause 27: The drug delivery device of any of clauses 19-26, wherein the adaptor is over-molded with the identification tag.
Clause 28: A drug delivery system, comprising: the drug delivery device of any of clauses 1-27; and a connector comprising an external surface comprising a thread, wherein the connector is engaged with the drug delivery device by the thread of the connector engaged with the internal thread of the adaptor.
Clause 29: The drug delivery system of clause 28, wherein the connector comprises an intravenous tube connector.
Clause 30: A method of manufacturing a drug delivery device of any of clauses 1-27, comprising: mounting the adaptor on the distal tip of the barrel; and applying the film to the at least a portion of the barrel and the adaptor.
Clause 31: The method of clause 30, further comprising sterilizing the drug delivery device prior to application of the film to the at least a portion of the barrel and the adaptor.
Clause 32: A method of delivering a composition with a drug delivery device of any of clauses 1-27, comprising: connecting the drug delivery device to a connector comprising an external surface comprising a thread, wherein the connector is engaged with the drug delivery device by the thread of the connector engaged with the internal thread of the adaptor; and causing the composition within an interior of the barrel to flow through the connector.

### BRIEF DESCRIPTION OF THE DRAWINGS

FIG. 1 is a cross section view of a drug delivery device according to non-limiting embodiments described herein;
FIG. 2 is a cross section view of a drug delivery device engaged with a plug according to non-limiting embodiments described herein;
FIG. 3 is a cross section view of a drug delivery device having an identification tag and engaged with a plug according to non-limiting embodiments described herein;
FIG. 4 is a perspective view of a drug delivery device engaged with a plug, prior to the drug delivery device being covered with a film according to non-limiting embodiments described herein;
FIG. 5 is a perspective view of a drug delivery device having an identification tag and engaged with a plug, prior to the drug delivery device being covered with a film according to non-limiting embodiments described herein;
FIG. 6 is a perspective view of a drug delivery device engaged with a plug and covered with a film, wherein a cut-away section is shown in the region of the adaptor according to non-limiting embodiments described herein;
FIG. 7 is a side view of a drug delivery device in which the film also covers part of a plug mounted on the adaptor, according to non-limiting embodiments described herein;
FIG. 8 is a perspective view of a film for the drug delivery device according to non-limiting embodiments described herein;
FIG. 9 is a perspective view of the film of FIG. 8, showing the film rolled up as if applied to the drug delivery device according to non-limiting embodiments described herein; and
FIG. 10 is a cross section view of a drug delivery device engaged with a connector according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

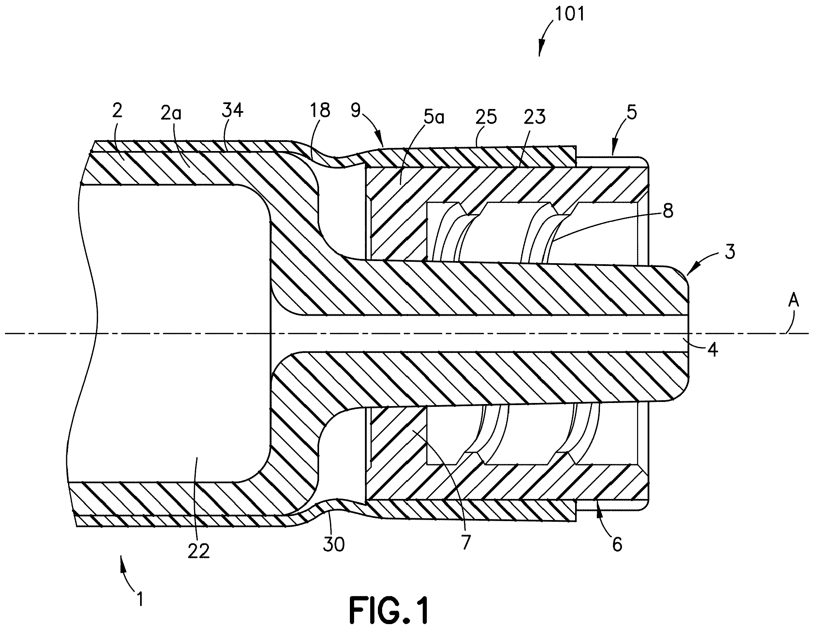

Referring to FIGS. 1-3, shown is a drug delivery device 1 of an assembly 101 according to some non-limiting embodiments. Drug delivery device 1 may include a barrel 2 defining an interior 22 configured to hold a composition. The composition may be a pharmaceutical composition. Drug delivery device 1 has a longitudinal axis A. Drug delivery device 1 may be a manual syringe, optionally a pre-filled syringe.

Barrel 2 may have a distal tip 3. Barrel 2 and distal tip 3 may be formed of one single material (e.g., plastic, glass, or other known materials). Barrel 2 and distal tip 3 may preferably be made of glass. Barrel 2 may be sealed at its proximal end by a piston (not shown). Distal tip 3 may define a channel 4 aligned with longitudinal axis A to provide a passageway for the transfer of the composition, either from barrel 2 to a connector 11 (not shown), such as an IV connector or an injection needle, or from a vial to barrel 2. The outer surface of the distal tip 3 may have a slightly tapered shape.

Referring to FIGS. 1-5, also shown is a cylindrical adaptor 5 defining a ring 6. Ring 6 may be provided in its proximal region with an inner projection in the form of a discontinuous annular bulge 7 extending radially inwardly. Annular bulge 7 may be made of a material flexible enough to allow annular bulge 7 to expand slightly radially in the outward direction under pressure exerted on the inner wall of annular bulge 7. Alternatively, annular bulge 7 may be replaced by a continuous annular bulge capable of expanding radially outwardly. The inner wall of ring 6 may be provided with an internal thread 8 defining at least a portion of an inner surface 23 of adaptor 5. Internal thread 8 may be distally spaced from annular bulge 7. Adaptor 5 may be made of flexible material such as plastic.

Adaptor 5 may be cylindrical in shape, as shown in FIGS. 1-5. The cylindrical shape of the adaptor 5 may have an outer surface 25 comprising at least one plane 28. The outer surface 25 may comprise a single plane. The outer surface 25 may comprise a pair of planes 28. The outer surface 25 may comprise more than 2 planes 28, such as 3 or 4 planes 28. In some non-limiting examples, a pair of planes 28 diametrically opposed (not shown) to one another may be included. By "diametrically opposed" it is meant that a first plane is arranged 40-60% around the circumference of the cylinder relative to a second plane. The first plane may be directly opposite the second plane (50% around the circumference of the cylinder). Planes 28 may be parallel to one another. The at least one plane 28 may run along a length of adaptor 5.

The cylindrical adaptor 5 may have a smooth outer surface 25. As used herein, "smooth" is meant to mean that outer surface 25 of adaptor 5 is free of significant projections of unevenness. A significant projection or unevenness is one in which one a diameter at one point of the cylindrical adaptor 5 is greater than a diameter at a second point of the cylindrical adaptor 5 by more than 5%. In some non-limiting embodiments (not shown), the cylindrical adaptor 5 may be perfectly cylindrical, having no projections or unevenness. The smooth outer surface 25 of adaptor 5 enables the adhesive film 9 to be more securely adhered thereto.

Adaptor 5 may be mounted on distal tip 3 by a friction fit, an adhesive, a threaded connection, or other connection known to those of skill in the art. Adaptor 5 may be rotatable relative to barrel 2 prior to application of film 9, described hereinafter. Adaptor 5 may abut a shoulder of barrel 2, such as being in physical contact therewith. Alternatively, a small gap may be present between adaptor 5 and the shoulder of barrel 2. The lack of gap or small gap between adaptor 5 and the shoulder of barrel 2 may form a more uniform cylinder suitable for secure adhesion of the adhesive film 9.

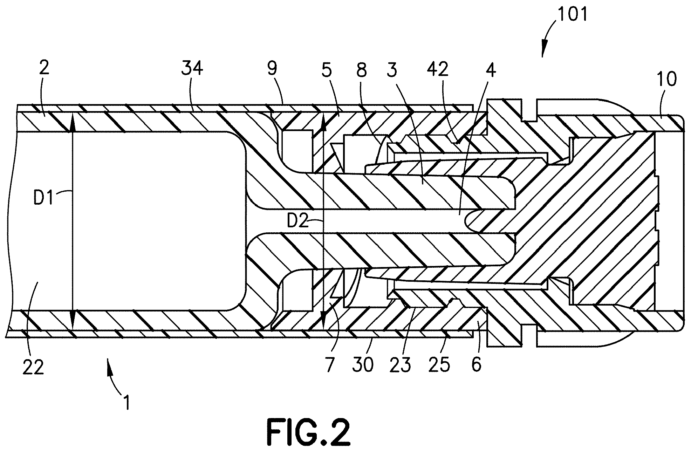

Referring to FIG. 2, a diameter D1 of barrel 2 may have substantially the same diameter as a diameter D2 of adaptor 5, such as a distal region 2a of barrel 2 having substantially the same diameter as a proximal region 5a of adaptor 5. Substantially the same diameter means the diameters D1, D2 are within 5%, such as within 1%, or identical to one another. Barrel 2 and adaptor 5 being substantially the same diameter may make drug delivery device 1 compatible for applying film 9 extending from barrel 2 to adaptor 5, as described hereinafter.

Referring again to FIGS. 1-3, shown is a non-limiting embodiment of drug delivery device 1 with adaptor 5 mounted on the distal tip 3. Drug delivery device 1 may be sterilized prior to application of film 9. Drug delivery device 1 may be steam sterilized or sterilized using any other process known to those of skill in the art. Film 9 may then be applied to sterilized drug delivery device 1, such as to at least a portion of barrel 2 and a portion of adaptor 5. Application of film 9 to both the barrel 2 and adaptor 5 may prevent rotation between adaptor 5 and barrel 2, such as during use of drug delivery device 1. Film 9 may be applied to distal region 2a of barrel 2 and proximal region 5a of adaptor 5, as shown in FIGS. 1-3.

Film 9 may comprise a label including one or more indicia. Film 9 may be transparent, translucent, or opaque and/or may comprise writing such as, for example, graduation, brand name, lot number, expiration date, drug code, or other useful information.

Film 9 may be made of a thermoplastic material. In some non-limiting embodiments, film 9 is made of paper, polyvinyl chloride, polyester, polyethylene, polyamide, polypropylene, vinyl, or some combination thereof. In some non-limiting embodiments, film 9 is made of a polyvinyl chloride-containing material

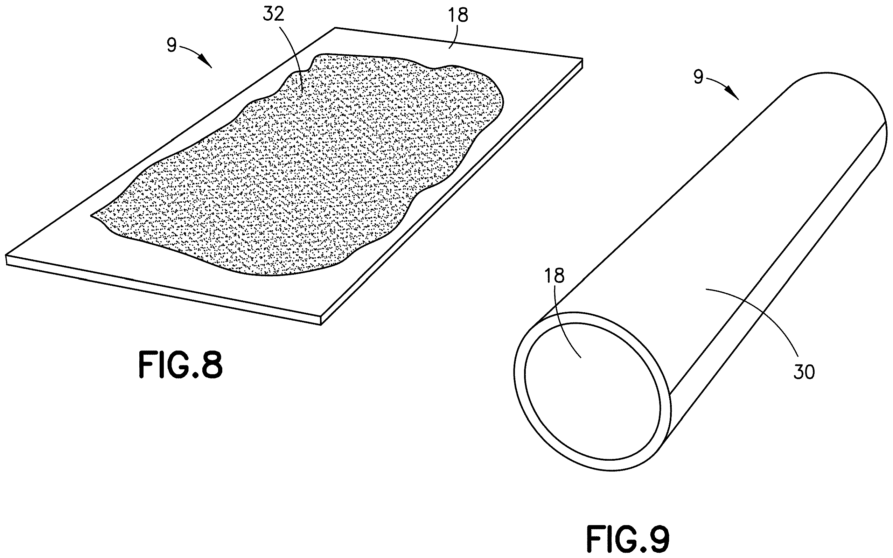

In some non-limiting embodiments, film 9 may be an adhesive film. As shown in FIGS. 8 and 9, film 9 may have an internal surface 18 and an external surface 30. Internal surface 18 of film 9 may have an adhesive material 32 over at least a portion thereof, as shown in FIG. 8. FIG. 9 shows film 9 rolled up as if applied to drug delivery device 1 (not shown). It will be appreciated that adhesive material 32 on internal surface 18 may adhere film 9 to barrel 2 and adaptor 5 when film 9 is rolled up and applied to drug delivery device 1. Adhesive material 32 may comprise a glue or be any other type of adhesive suitable for fixedly adhering film 9 to barrel 2 and adaptor 5, thereby preventing relative rotation between adaptor 5 and the barrel 2.

As can be appreciated from FIGS. 1-3, adaptor 5 may be limited and/or prevented from rotating with respect to barrel 2 around longitudinal axis A by the presence of film 9. Film 9 may also prevent the adaptor 5 from translating lengthwise with respect to barrel 2. As a result, when a user proceeds to attach drug delivery device 1 including adaptor 5 to another device (e.g., connector 11), the operation is facilitated and secure.

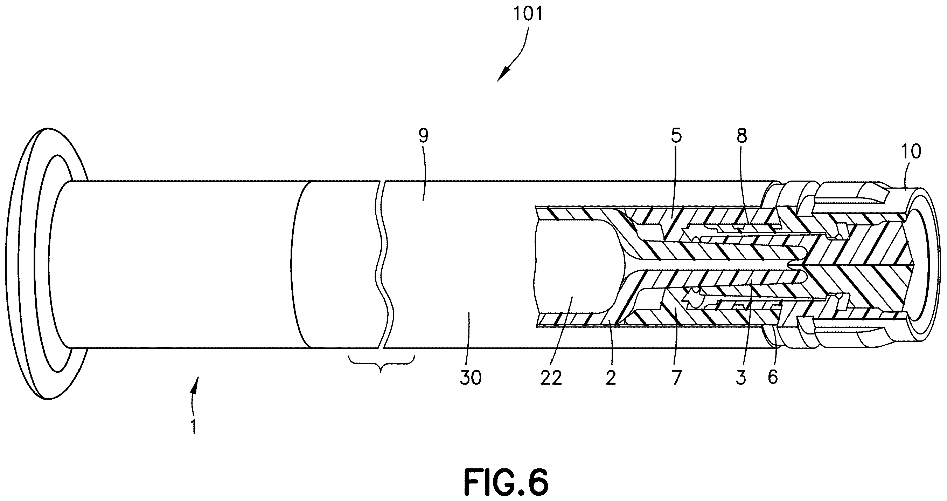

Referring to FIGS. 2-7, drug delivery device 1 may comprise a plug 10 mounted on adaptor 5. Plug 10 may be removably connected to adaptor 5, and may limit access to adaptor 5 before use, in order to protect adaptor 5 and/or maintain sterility of adaptor 5. Plug 10 may comprise threads 42 configured to engage with internal thread 8 of adaptor 5 to engage plug 10 with adaptor 5 in a secure screw connection.

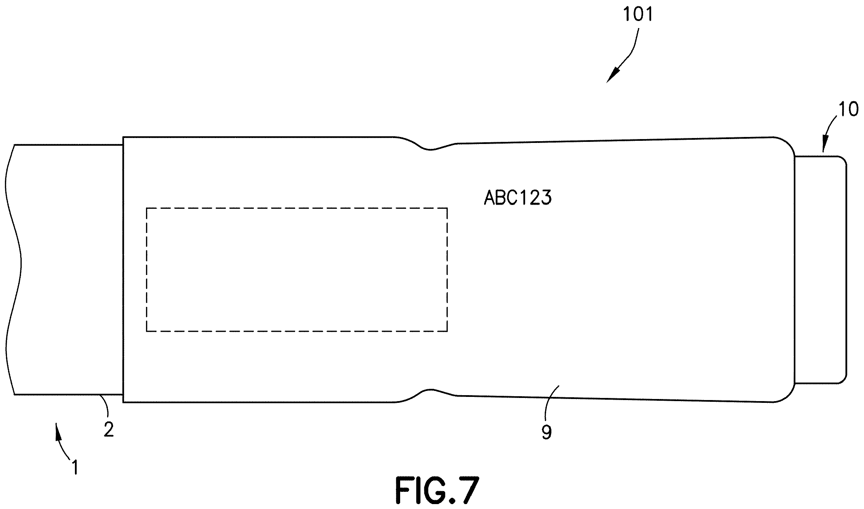

Referring to FIG. 7, shown is a non-limiting embodiment, in which film 9 is longer and covers at least a portion of plug 10. Film 9 may therefore cover a portion of barrel 2, adaptor 5 (not shown) and a portion of plug 10, such as in a continuous manner. In non-limiting embodiments in which film 9 also covers a portion of plug 10 mounted on adaptor 5, plug 10 may be mounted on adaptor 5 before applying film 9, and the length of film 9 may be chosen so as to cover adaptor 5 and at least a portion of plug 10.

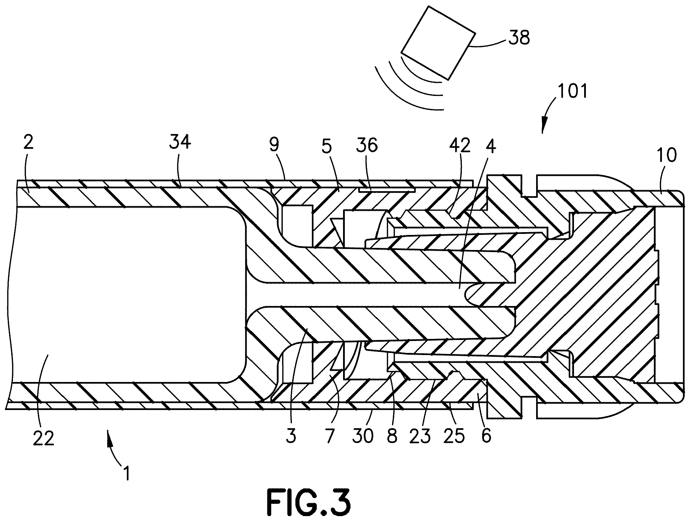

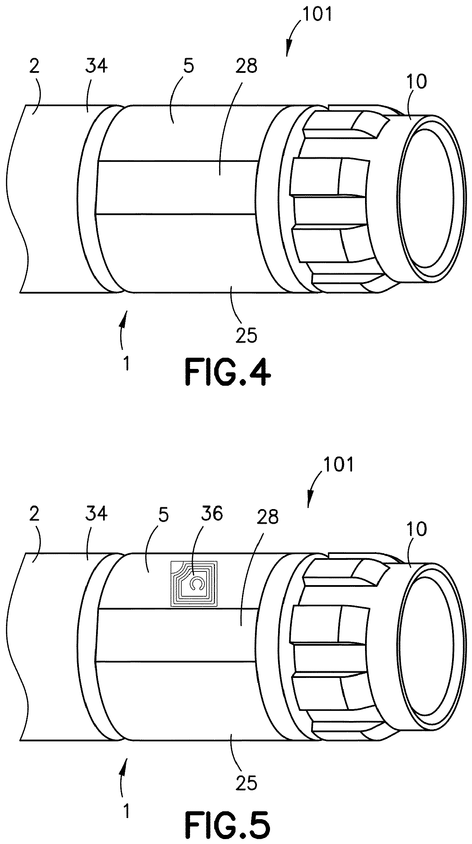

Referring to FIGS. 3 and 5, drug delivery device 1 may further comprise an identification tag 36. Identification tag 36 may be arranged with adaptor 5 and beneath film 9. Identification tag 36 may be configured to communicate wirelessly with a corresponding reader device 38. Identification tag 36 may be arranged on outer surface 25 of adaptor 5 and beneath film 9. Identification tag 36 may be embedded in a wall of adaptor 5 and beneath film 9. Identification tag 36 may be over-molded over adaptor 5 and beneath film 9. Identification tag 36 may be arranged beneath film 9 so as to be invisible from the user during use of drug delivery device 1.

Identification tag 36 may store data about drug delivery device 1. For example, the data may comprise a unique identifier to allow for identification and/or traceability of a specific drug delivery device 1. The data may comprise other useful data, such as type of composition contained in drug delivery device 1, dosage of the composition, expiration date of composition, preparation date of composition, intended recipient information, administration instructions, side effect information, and the like. The data may include historic location data (e.g., location and date/time) of drug delivery device 1 to trace historical location of drug delivery device 1.

Identification tag 36 may communicate wirelessly with reader device 38. In some non-limiting examples, identification tag 36 is a radio frequency identification (RFID) tag, and reader device 38 is an RFID reader. However, it will be appreciated that other wireless and/or short-range communication technologies may be used for identification tag 36 and reader device 38. Reader device 38 may communicate with identification tag 36 to read the data stored on identification tag 36. Reader device 38 may display at least a portion of the read data on a user interface (not shown) such that a user may view the data stored on identification tag 36. Reader device 38 communicating with identification tag 36 may cause updated location data associated with drug delivery device 1 to be stored, such as in a central database tracking location of a plurality of drug delivery devices.

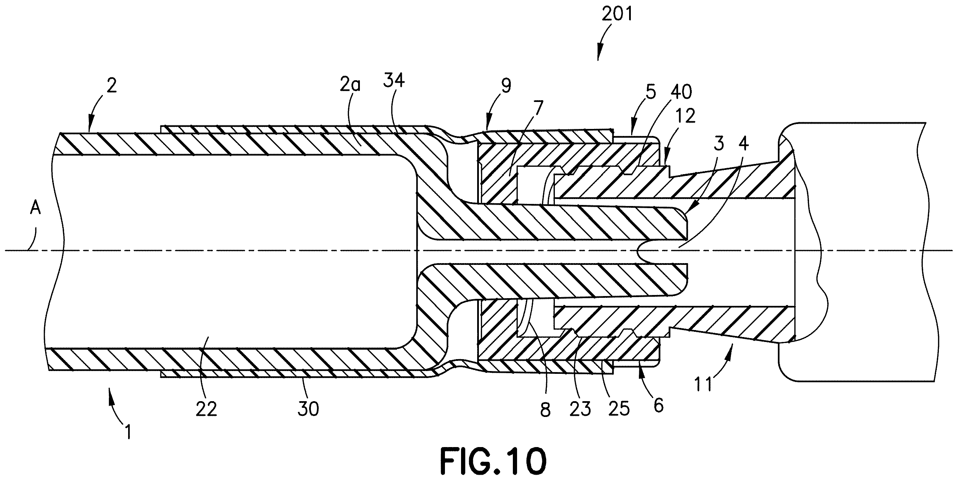

Referring to FIG. 10, a drug delivery system 201 is shown according to some non-limiting embodiments. Drug delivery system 201 may comprise drug delivery device 1 as described herein. Drug delivery system 201 may also comprise a connector 11. Connector 11 may be any other device having a connection compatible with adaptor 5. For example, connector 11 may comprise a connector to an IV, an injection needle, or the like. Connector 11 may have a luer connection. Connector 11 may be provided with an outer thread 12 on an external surface 40 of connector 11. Outer thread 12 may be configured to match internal thread 8 of adaptor 5 to engage connector 11 with adaptor 5 in a secure screw connection. Drug delivery device 1 may engage with connector 11 after application of film 9 to barrel 2 and adaptor 5.

Referring back to FIGS. 2-7 and 10, shown are non-limiting embodiments of drug delivery device 1 with a closed adaptor 5. In FIGS. 2-7, adaptor 5 is closed by engagement with plug 10, and in FIG. 10, adaptor 5 is closed by engagement with connector 11. To begin the process of connecting adaptor 5 closed by plug 10 to another device, such as connector 11, the user may first grasp the drug delivery device 1 via the film 9 in one hand to disengage plug 10. The user may then attach connector 11 onto internal thread 8 of adaptor 5 to engage connector 11 with adaptor 5, as shown in FIG. 10, without rotation or lengthwise translation of adaptor 5 relative to barrel 2 due to the presence of film 9. When the outer thread 12 of connector 11 is firmly received by internal thread 8 of adaptor 5, drug delivery device 1 and connector 11 may be tightly connected preventing leakage of the composition when flowed from or to barrel 2.

Drug delivery device 1, as described herein, may be manufactured by mounting adaptor 5 on distal tip 3 of barrel 2. Barrel 2 may be pre-filled with a pharmaceutical composition. Film 9 may be applied to at least a portion of barrel 2 and a portion of adaptor 5, thereby preventing relative rotation between adaptor 5 and barrel 2. Film 9 may be fixedly adhered to barrel 2 and adaptor 5 by adhesive material 32 on internal surface 18 of film 9 contacting barrel 2 and adaptor 5.

Drug delivery device 1, as described herein, may deliver a composition contained in barrel 2, such as to a patient or to another device. Drug delivery device 1 may deliver the composition by connecting drug delivery device 1 to connector 11 comprising external surface 40 comprising outer thread 12, wherein connector 11 is engaged with drug delivery device 1 by outer thread 12 of connector 11 engaged with internal thread 8 of the adaptor 5. The method may further include causing the composition within interior 22 of barrel 2 to flow through the connector 11. For example, drug delivery device 1 may comprise a plunger (not shown) comprising the piston at the proximal end forcing the composition from barrel 2 through channel 4, through adaptor 5, through connector 11.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A drug delivery device, comprising:
a barrel defining an interior configured to receive a composition, the barrel comprising a distal tip defining a channel to provide a passageway for transfer of the composition;
a cylindrical adaptor mounted onto the distal tip, the adaptor comprising an internal thread defining at least a portion of an inner surface and an outer surface comprising at least one plane, the adaptor rotatable relative to the barrel; and
a film applied to at least a portion of the barrel and at least a portion of the adaptor, thereby preventing relative rotation between the adaptor and the barrel.

2. The drug delivery device of claim 1, wherein the drug delivery device is sterilized prior to application of the film to the at least a portion of the barrel and the adaptor.

3. The drug delivery device of claim 1 or 2, wherein the film comprises an inner surface and an opposed outer surface, wherein at least a portion of the inner surface comprises an adhesive, such that when the film is applied to the at least a portion of the barrel and the adaptor, the inner surface of the film is adhered to a surface of the barrel and a surface of the adaptor.

4. The drug delivery device of any of claims 1-3, further comprising a plug removably connected to the adaptor.

5. The drug delivery device of any of claims 1-4, wherein a diameter of the barrel is substantially the same as a diameter of the adaptor.

6. The drug delivery device of any of claims 1-5, wherein the barrel comprises glass.

7. The drug delivery device of any of claims 1-6, wherein the outer surface of the adaptor is smooth.

8. The drug delivery device of any of claims 1-7, further comprising an identification tag arranged with the adaptor and beneath the film, wherein the identification tag is configured to communicate wirelessly with a corresponding reader device.

9. The drug delivery device of claim 8, wherein the adaptor is over-molded with the identification tag.

10. A drug delivery system, comprising:
the drug delivery device of any of claims 1-9; and
a connector comprising an external surface comprising a thread,
wherein the connector is engaged with the drug delivery device by the thread of the connector engaged with the internal thread of the adaptor.

11. The drug delivery system of claim 10, wherein the connector comprises an intravenous tube connector.

12. A method of manufacturing a drug delivery device of any of claims 1-9, comprising:
mounting the adaptor on the distal tip of the barrel; and
applying the film to the at least a portion of the barrel and the adaptor.

13. The method of claim 12, further comprising:
sterilizing the drug delivery device prior to application of the film to the at least a portion of the barrel and the adaptor.

14. A method of delivering a composition with a drug delivery device of any of claims 1-9, comprising:
connecting the drug delivery device to a connector comprising an external surface comprising a thread, wherein the connector is engaged with the drug delivery device by the thread of the connector engaged with the internal thread of the adaptor; and
causing the composition within an interior of the barrel to flow through the connector.
